(19) Europäisches Patentamt
European Patent Office
Office européen des brevets

(11) Veröffentlichungsnummer: **0 053 695**
**A2**

(12) # EUROPÄISCHE PATENTANMELDUNG

(21) Anmeldenummer: **81108812.9**

(22) Anmeldetag: **23.10.81**

(51) Int. Cl.³: **C 07 D 239/49**
A 61 K 31/505

(30) Priorität: **04.12.80 DE 3045720**

(43) Veröffentlichungstag der Anmeldung:
**16.06.82 Patentblatt 82/24**

(84) Benannte Vertragsstaaten:
**AT BE CH DE FR GB IT LI LU NL SE**

(71) Anmelder: **BASF Aktiengesellschaft**
**Carl-Bosch-Strasse 38**
**D-6700 Ludwigshafen(DE)**

(72) Erfinder: **Scharwaechter, Peter, Dr.**
**Klinkerstrasse 26**
**D-2082 Moorrege(DE)**

(72) Erfinder: **Gutsche, Klaus, Dr.**
**Ehmsenkamp 5**
**D-2084 Rellingen(DE)**

(72) Erfinder: **Kohlmann, Friedrich-Wilhelm, Dr.**
**An der Duene 6**
**D-2082 Moorrege(DE)**

(72) Erfinder: **Beck, Hinrich, Dr.**
**Lieth 9**
**D-2357 Bad Bramstedt(DE)**

(54) **N-Pyrimidinyl-carbaminsäureester, Verfahren zu ihrer Herstellung und diese enthaltende Arzneimittel.**

(57) Die Erfindung betrifft 2-Carbaminsäureester des 2,4-Diamino-5-(3′,4′,5′-trimethoxybenzyl)-pyrimidins (Trimethoprims), Verfahren zu ihrer Herstellung und diese enthaltende Arzneimittel, die als Prodrugs des Trimethoprims wertvolle antibakterielle Eigenschaften aufweisen und die antibakterielle Wirkung von Sulfonamiden potenzieren.

EP 0 053 695 A2

N-Pyrimidinyl-carbaminsäureester, Verfahren zu ihrer Herstellung und diese enthaltende Arzneimittel

Es ist bekannt, daß substituierte 5-Benzyl-pyrimidine wertvolle Arzneimittel darstellen. So wird beispielsweise das 2,4-Diamino-5-(3,4,5-trimethoxybenzyl)-pyrimidin, auch Trimethoprim genannt, als antibakteriell wirksame Verbindung erfolgreich in der Humanmedizin eingesetzt, obwohl es nicht immer allen gewünschten Anforderungen,u.a. wegen der relativ kurzen Wirkdauer, entspricht.

Weitere Benzylpyrimidine, beispielsweise 4-Amido-2-amino-5--(3,4,5-trimethoxybenzyl)-pyrimidine, werden in der DE-OS 27 09 634 oder der BE-PS 850 974 beschrieben.

Es wurde nun gefunden, daß neue N-Pyrimidinylcarbaminsäureester der Formel I

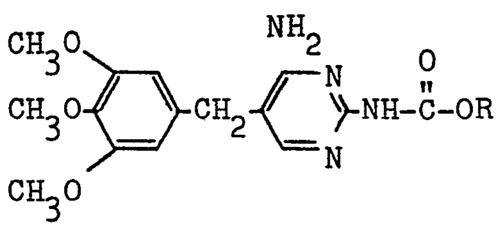

(I),

in der R einen Alkylrest mit 1 bis 4 C-Atomen, der durch einen Phenylrest oder durch eine Alkoxygruppe mit 1 bis 3 C-Atomen im Alkyl, die mindestens 2 C-Atome vom Carbaminsäureestersauerstoff entfernt ist, substituiert sein kann, bedeutet, und ihre pharmakologisch verträglichen Salze als Prodrugs des Trimethoprims wertvolle antibakterielle Eigenschaften mit verlängerter Wirkungsdauer im Vergleich zum Trimethoprim aufweisen und die antibakterielle Wirkung von Sulfonamiden potenzieren, wodurch eine insgesamt verbesserte Therapie gegen zu behandelnde bakterielle Infektion erreicht werden kann.
Als Reste für R sind beispielsweise zu nennen Methyl, Ethyl, Isopropyl, Butyl, (2-Methoxy)-ethyl, Benzyl.

Davon sind Ethyl und (2-Methoxy)-ethyl besonders hervorzuheben.

Die Herstellung der erfindungsgemäßen Verbindungen der Formel I wird nach einer für die Herstellung von Carbaminsäureestern an sich üblichen Methode durchgeführt, indem man eine Verbindung der Formel II

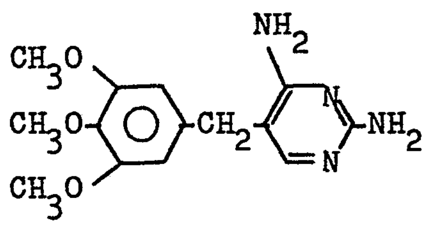 (II)

mit einem Chlorameisensäureester der Formel III

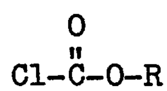 (III) ,

in der R die für Formel I angegebenen Bedeutungen hat, gegebenenfalls in Gegenwart eines Lösungsmittels und gegebenenfalls eines säurebindenden Mittels umsetzt.

Als Lösungsmittel für die Umsetzung sind cyclische gesättigte Ether, wie Dioxan oder Tetrahydrofuran, aliphatische chlorierte Kohlenwasserstoffe, wie Chloroform, zweckmäßig. Davon ist Tetrahydrofuran besonders bevorzugt. Der bevorzugte Temperaturbereich liegt bei Temperaturen von 0 bis 50°C.

Als säurebindende Mittel kommen tertiäre organische Basen, wie beispielsweise Triethylamin oder Pyridin, oder Alkalimetallhydroxide, insbesondere Natriumhydroxid, in Betracht. Gegebenenfalls wird eine zu verwendende tertiäre organische Base in überschüssiger Menge als Lösungsmittel

verwendet. Für die Herstellung von Säureadditionssalzen, insbesondere von in pharmazeutischen Präparaten brauchbaren Salzen, kommen die üblicherweise für diesen Zweck verwendeten anorganischen Säuren, wie Salzsäure, Schwefelsäure, Phosphorsäure etc. oder organische Säuren, wie Ameisensäure, Essigsäure, Milchsäure etc. in Betracht oder können beispielsweise dem Journal of Pharmaceuticals Sciences, Volume 66, Seiten 1 bis 5 (1977) entnommen werden.

Die erfindungsgemäßen Verbindungen der Formel I und ihre Salze sind antibakteriell wirksam und potenzieren, kombiniert mit antibakteriell wirksamen Sulfonamiden, deren antibakterielle Wirkungen. Solche Sulfonamide sind beispielsweise Sulfadiazin, Sulfamethoxin, Sulfadimethoxin, Sulfamethoxazol, Sulfamoxol, 2-Sulfa-4,5-dimethyl--isoxazol, 4-Sulfanilamido-5,6-dimethoxy-pyrimidin. Sie können daher beispielsweise bei bakteriellen Erkrankungen der Atmungsorgane, Verdauungsorgane und Harnwege sowie bei Hals-, Nasen-, Ohreninfektionen und allgemein systemischen Infektionskrankheiten verwendet werden.

Die Verbindungen der Formel I können mit den beispielhaft genannten Sulfonamiden in verschiedenen Mischungsverhältnissen kombiniert werden, wobei das Verhältnis Verbindung der Formel I zu Sulfonamid in dem Bereich von 1 : 5 bis 5 : 1 variieren kann; bevorzugte Verhältnisse sind 1 : 1 bis 1 : 5. Dabei kommen in der Regel als Einzeldosierung 20 bis 500 mg eines Wirkstoffs der Formel I in Betracht.

Gegenstand der vorliegenden Erfindung ist demnach auch eine pharmazeutische Zubereitung bzw. ein chemotherapeutisches Mittel, enthaltend eine Verbindung der Formel I oder deren pharmakologisch verträgliches Salz gemäß Anspruch 1, gegebenenfalls zusammen mit einem antibakteriell

wirksamen Sulfonamid, und nicht-toxischen, therapeutisch verträglichen festen oder flüssigen Trägerstoffen und galenischen Hilfsmitteln, sowie die Verwendung der Verbindungen der Formel I als Sulfonamidpotentiatoren.

Die pharmazeutischen Mittel bzw. Zubereitungen werden mit den üblichen nicht-toxischen, therapeutisch verträglichen, festen oder flüssigen Trägerstoffen und den üblicherweise verwendeten pharmazeutisch-technischen Hilfsstoffen entsprechend der gewünschten Applikationsart in bekannter Weise hergestellt (vgl. L.G. Godman, A. Gilman, The Pharmacological Basis of Therapeutics).

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen.

Zum Nachweis der verlängerten antibakteriellen Wirksamkeit wurden ca. 200 g schwere Sprague-Dawley-Ratten einmal mit 40 mg/kg Trimethoprim oder mit der äquimolaren Menge der zu prüfenden Substanz oral behandelt und nach 1, 3, 5 und 7 Stunden durch Herzpunktion entblutet. Der Wirkstoffgehalt im Serum wurde im Agardiffusionstest mit Bacillus pumilus als Indikatororganismus ermittelt (Bushley, S.R.M. und Hitchings, G.H., Bri.J.Pharmac. Chemother. (1968), 33, 72 bis 90) und die Konzentrationsbestimmung an Hand der Hemmhofgrößen von unter gleichen Versuchsbedingungen erstellten Trimethoprim-Standards graphisch (Klein, P., "Bakteriologische Grundlagen der chemotherap. Laboratoriumspraxis", Springer-Verlag) und rechnerisch (J.V. Bennett el al., Appl. Microbiol. (1966), 14, 170 bis 177) vorgenommen und auf Trimethoprim bezogen.

Die Tabelle 1 zeigt das Ergebnis dieser Untersuchungen. Danach zeigen die Substanzbeispiele 1 und 2 gegenüber Trimethoprim (TMP) eine Verlängerung der Verweilzeit des mikrobiologisch aktiven Gehaltes im Serum bis zum Dreifachen.

Tabelle 1

Antibakteriell wirksamer Gehalt im Serum von Ratten nach Verabreichung von 40 mg/kg Trimethoprim (TMP) oder der äquimolaren Menge der Verbindung von Beispiel 1 oder 2

| Beispiel | Serumspiegel (µg TMP/ml) ... Stunden nach Applikation | | | |
|---|---|---|---|---|
| | 1 | 3 | 5 | 7 |
| 1 | 1,89 | 1,75 | 1,34 | 0,54 |
| 2 | 2,0 | 1,23 | 0,62 | 0,33 |
| TMP | 6,34 | 2,5 | 0,72 | 0,18 |

Die vorliegende Erfindung wird durch die folgenden Ausführungsbeispiele näher erläutert.

Beispiel 1

Zu einer Suspension von 11,6 g 2,4-Diamino-5-(3,4,5-trimethoxybenzyl)-pyrimidin (Trimethoprim) in 80 ml Tetrahydrofuran und 5,56 ml Triäthylamin werden zwischen 3 bis 5°C unter Rührung 3,81 ml Chlorameisensäureethylester, gelöst in 20 ml Tetrahydrofuran, tropfenweise zugegeben. Nach 10-stündigem Rühren bei Raumtemperatur wird der Niederschlag abgetrennt und mit Wasser gewaschen. Nach Lösen in Dimethylformamid und Versetzen mit Wasser erhält man 12,1 g (84,1 % der Theorie) kristallines N-[4-Amino-5-(3,4,5-trimethoxybenzyl)]-pyrimidin-2-yl-carbaminsäure--ethylester mit dem Schmelzpunkt 205 - 208°C.
$C_{17}H_{22}N_4O_5$ (362,38)

| | | | | | | |
|---|---|---|---|---|---|---|
| Ber.: | C | 56,34 % | H | 6,12 % | N | 15,46 % |
| Gef.: | | 56.2 % | | 6,2 % | | 15,3 % |

Beispiel 2

3,62 g N-[4-Amino-5-(3,4,5-trimethoxybenzyl)]-pyrimidin-2--yl-carbaminsäure-ethylester werden in 75 ml Chloroform bei Siedetemperatur gelöst und unter Rühren tropfenweise mit 30 ml gesättigter Diisopropylether/Chlorwasserstoff--Lösung versetzt. Nach dem Verreiben des öligen Niederschlages mit Diisopropylether erhält man 3,1 g kristallines N-[4-Amino-5-(3,4,5-trimethoxybenzyl)-pyrimidin-2-yl]-carbaminsäure-ethylester-hydrochlorid mit dem Schmelzpunkt 170°C (Z.).
$C_{17}H_{23}ClN_4O_5$ (398,86)

| | | | | | | | | |
|---|---|---|---|---|---|---|---|---|
| Ber.: | C | 51,19 % | H | 5,81 % | N | 14,05 % | Cl | 8,89 % |
| Gef.: | | 50,9 % | | 5,85 % | | 14,5 % | | 8,6 % |

Analog zum Beispiel 1 werden synthetisiert:

Beispiel 3

N-[4-Amino-5-(3,4,5-trimethoxybenzyl)]-pyrimidin-2-yl--carbaminsäure-methylester aus Chlorameisensäure-methylester und 2,4-Diamino-5-(3,4,5-trimethoxybenzyl)-pyrimidin mit dem Schmelzpunkt 200°C.
$C_{16}H_{20}N_4O_5$ (348,35).

| | | | | | | |
|---|---|---|---|---|---|---|
| Ber.: | C | 55,16 % | H | 5,79 % | N | 16,08 % |
| Gef.: | | 54,9 % | | 5,8 % | | 16,0 % |

Beispiel 4

N-[4-Amino-5-(3,4,5-trimethoxybenzyl)]-pyrimidin-2-yl--carbaminsäure-(2-methoxy)-ethylester aus Chlorameisensäure-(2-methoxy)-ethylester und 2,4-Diamino-5-(3,4,5-trimethoxybenzyl)-pyrimidin mit dem Schmelzpunkt 211 bis 215°C.

$C_{18}H_{24}N_4O_6$ (392,4)

| | | | | | | |
|---|---|---|---|---|---|---|
| Ber.: | C | 55,09 % | H | 6,17 % | N | 14,28 % |
| Gef.: | | 54,6 % | | 6,2 % | | 14,3 % |

Beispiel 5

N-[4-Amino-5-(3,4,5-trimethoxybenzyl)]-pyrimidin-2-yl--carbaminsäure-isopropylester aus Chlorameisensäure-isopropylester und 2,4-Diamino-5-(3,4,5-trimethoxybenzyl)--pyrimidin mit dem Schmelzpunkt 192 bis 195°C.

$C_{18}H_{24}N_4O_5$ (376,4)

| | | | | | | |
|---|---|---|---|---|---|---|
| Ber.: | C | 57,43 % | H | 6,43 % | N | 14,89 % |
| Gef.: | | 56,9 % | | 6,3 % | | 14,8 % |

Beispiel 6

N-[4-Amino-5-(3,4,5-trimethoxybenzyl)]-pyrimidin-2-yl--carbaminsäure-butylester aus Chlorameisensäure-butylester und 2,4-Diamino-5-(3,4,5-trimethoxybenzyl)-pyrimidin mit dem Schmelzpunkt 206°C.

$C_{19}H_{26}N_4O_5$ (390,43)

| | | | | | | |
|---|---|---|---|---|---|---|
| Ber.: | C | 58,45 % | H | 6,71 % | N | 14,35 % |
| Gef.: | | 57,9 % | | 6,7 % | | 14,4 % |

Beispiel 7

N-[4-Amino-5-(3,4,5-trimethoxybenzyl)]-pyrimidin-2-yl--carbaminsäure-isobutylester aus Chlorameisensäure-isobutylester und 2,4-Diamino-5-(3,4,5-trimethoxybenzyl)--pyrimidin mit dem Schmelzpunkt 198°C.
$C_{19}H_{26}N_4O_5$ (390,43)

| | | | | | | |
|---|---|---|---|---|---|---|
| Ber.: | C | 58,45 % | H | 6,71 % | N | 14,35 % |
| Gef.: | | 57,9 % | | 6,74 % | | 14,54 % |

Beispiel 8

N-[4-Amino-5-(3,4,5-trimethoxybenzyl)]-pyrimidin-2-yl--carbaminsäure-benzylester aus Chlorameisensäure-benzylester und 2,4-Diamino-5-(3,4,5-trimethoxybenzyl)-pyrimidin mit dem Schmelzpunkt 240°C.
$C_{22}H_{24}N_4O_5$ (424,44)

| | | | | | | |
|---|---|---|---|---|---|---|
| Ber.: | C | 62,25 % | H | 5,70 % | N | 13,20 % |
| Gef.: | | 61,8 % | | 5,7 % | | 12,3 % |

Beispiele für Zubereitungen

Beispiel 9

400 mg 2-Sulfanilamido-5-methoxy-pyrimidin (Sulfamethoxydiazin)
80 mg N-[4-Amino-5-(3,4,5-trimethoxybenzyl)]-pyrimidin-2--yl-carbaminsäure-ethylester
20 mg Maisstärke
10 mg Gelatine
8 mg Talkum
2 mg Magnesiumstearat
20 mg Primojel

Die Wirkstoffe werden mit Maisstärke gemischt und mit wäßriger Gelatinelösung granuliert. Das trockene Granulat wird gesiebt und mit den Zuschlägen vermischt. Aus dieser Mischung werden in üblicher Weise Tabletten gepreßt.

Beispiel 10

250 mg 2-Sulfanilamido-5-methoxy-pyrimidin (Sulfamethoxydiazin)
100 mg N-[4-Amino-5-(3,4,5-trimethoxybenzyl)]-pyrimidin--2-yl-carbaminsäureethylester
5 mg Gelatine
30 mg Maisstärke
4 mg Talkum
1 mg Magnesiumstearat

Die Wirkstoffe werden mit wäßriger Gelatinelösung granuliert und nach dem Trocknen mit Maisstärke, Talkum und Magnesiumstearat vermischt. Aus dieser Mischung werden in üblicher Weise Tabletten gepreßt.

Beispiel 11

400 mg 2-Sulfanilamidopyrimidin (Sulfadiazin)
80 mg N-(4-Amino-5-(3,4,5-trimethoxybenzyl)]-pyrimidin--2-yl-carbaminsäureethylester
20 mg Maisstärke
10 mg Gelatine
8 mg Talkum
2 mg Magnesiumstearat
20 mg Primojel

Die Wirkstoffe werden mit Maisstärke gemischt und mit wäßriger Gelatinelösung granuliert. Das trockene Granulat

wird gesiebt und mit den Zuschlägen vermischt. Aus dieser Mischung werden in üblicher Weise Tabletten gepreßt.

Beispiel 12

250 mg 2-Sulfanilamido-pyrimidin (Sulfadiazin)
100 mg N-[4-Amino-5-(3,4,5-trimethoxybenzyl)]-pyrimidin--2-yl-carbaminsäure-ethylester
1,9 g Tylose C 30
30,0 g Zucker
10,0 g Glycerin
2,5 g Bentonit
0,06g Aroma
0,04g Nipagin M
0,06g Nipasol-Natrium
ad 100,000 g demineralisiertes Wasser

Die feinst gemahlenen Wirkstoffe werden in dem wäßrigen Tylose-Schleim suspendiert. Anschließend werden alle anderen Bestandteile unter Rühren nacheinander zugegeben. Zum Schluß wird mit Wasser auf 100,0 g aufgefüllt.

Patentansprüche

1. N-Pyrimidinyl-carbaminsäureester der allgemeinen Formel I

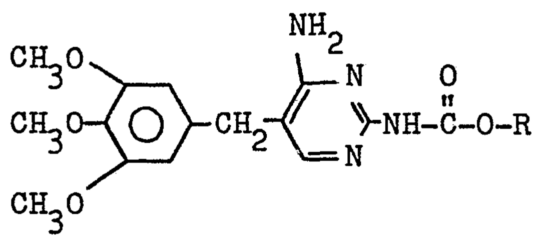

(I) ,

in der R einen Alkylrest mit 1 bis 4 C-Atomen, der durch einen Phenylrest oder durch eine Alkoxygruppe mit 1 bis 3 C-Atomen im Alkyl, die mindestens 2 C-Atome vom Carbaminsäureestersauerstoff entfernt ist, substituiert sein kann, bedeutet, sowie deren pharmakologisch verträgliche Salze.

2. N-[4-Amino-5-(3,4,5-trimethoxybenzyl)]-pyrimidin-2-yl-carbaminsäure-ethylester sowie seine pharmakologisch verträglichen Salze.

3. N-[4-Amino-5-(3,4,5-trimethoxybenzyl)]-pyrimidin-2-yl-carbaminsäure-(2-methoxy)-ethylester sowie seine pharmakologisch verträglichen Salze.

4. N-[4-Amino-5-(3,4,5-trimethoxybenzyl)]-pyrimidin-2-yl-carbaminsäure-benzylester sowie seine pharmakologisch verträglichen Salze.

5. Verfahren zur Herstellung der Verbindungen der Formel I nach Anspruch 1, dadurch gekennzeichnet, daß man eine Verbindung der Formel II

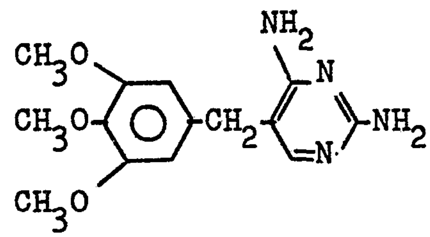

(II)

mit einem Chlorameisensäureester der Formel III

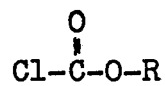

(III) ,

in der R die für Formel I angegebenen Bedeutungen hat, gegebenenfalls in Gegenwart eines Lösungsmittels und eines säurebindenden Mittels umsetzt und gegebenenfalls in das Salz einer pharmakologisch verträglichen Säure überführt.

6. Pharmazeutische Zubereitung, enthaltend eine Verbindung der Formel I oder deren pharmakologisch verträgliches Salz gemäß Anspruch 1, gegebenenfalls zusammen mit einem antibakteriell wirksamen Sulfonamid, und nicht-toxischen, therapeutisch verträglichen festen oder flüssigen Trägerstoffen und galenischen Hilfsmitteln.